# EUROPEAN PATENT SPECIFICATION

(11) **EP 4 282 365 B1**
(45) Date of publication and mention of the grant of the patent: **18.11.2026**
(21) Application number: 23175329.4
(22) Date of filing: 25.05.2023
(51) Int. Cl.: A61B 18/14, A61B 90/00

(54) **PORT AND DEPTH STOP AND TIP FEATURES FOR TRANSSEPTAL PUNCTURE APPARATUS**
ÖFFNUNGS- UND TIEFENANSCHLAGS- UND SPITZENFUNKTIONEN FÜR EINE TRANSSEPTALE PUNKTIONSVORRICHTUNG
ÉLÉMENTS D'ORIFICE ET D'ARRÊT DE PROFONDEUR ET DE POINTE POUR APPAREIL DE PONCTION TRANSSEPTALE

(30) Priority: 26.05.2022 US 202217825233
(43) Date of publication of application: 29.11.2023
(73) Proprietor: BIOSENSE WEBSTER (ISRAEL) LTD., 2066717 Yokneam (IL)
(72) Inventor: HIGHSMITH, Debby E., Irvine, 92618 (US)
(74) Representative: Carpmaels & Ransford LLP

(56) References cited:
- WO-A1-2022/001670
- WO-A1-2022/046777
- US-A1- 2010 168 728
- US-A1- 2016 374 751
- US-A1- 2022 032 007

## Description

### BACKGROUND

Cardiac arrhythmias, such as atrial fibrillation, occur when regions of cardiac tissue abnormally conduct electric signals. Procedures for treating arrhythmia include surgically disrupting the conducting pathway for such signals. By selectively ablating cardiac tissue by application of electrical energy (e.g., radiofrequency (AC type) or pulsed field (DC type) energy), it may be possible to cease or modify the propagation of unwanted electrical signals from one portion of the heart to another. The ablation process may provide a barrier to unwanted electrical pathways by creating electrically insulative lesions or scar tissue that effectively block communication of aberrant electrical signals across the tissue.

In some procedures, a catheter with one or more electrodes may be used to provide ablation within the cardiovascular system. The catheter may be inserted into a major vein or artery (e.g., the femoral artery) and then advanced to position the electrodes within the heart or in a cardiovascular structure adjacent to the heart (e.g., the pulmonary vein). The one or more electrodes may be placed in contact with cardiac tissue or other vascular tissue and then activated with electrical energy to thereby ablate the contacted tissue. In some cases, the electrodes may be bipolar. In some other cases, a monopolar electrode may be used in conjunction with a ground pad or other reference electrode that is in contact with the patient that is in contact with the patient. Irrigation may be used to draw heat from ablating components of an ablation catheter; and to prevent the formation of blood clots near the ablation site.

In some instances, it may be desirable to traverse the atrial septum to facilitate access various types of cardiac interventions in the left atrium of the heart, including electrophysiological or structural testing, treatment (e.g., ablation), or mapping. To achieve traversal of the atrial septum, a needle or other instrument may be used to form an opening through the septum. Examples of transseptal needles and associated components are disclosed in U.S. Pat. No 6,994,094, entitled "Method and Device for Transseptal Facilitation Based on Injury Patterns," issued February 7, 2006; U.S. Pat. No 8,152,829, entitled "Retractable Dilator Needle," issued April 10, 2012; U.S. Pat. No 9,848,943, entitled "Ablation Catheter with Dedicated Fluid Paths and Needle Centering Insert," issued December 26, 2017; U.S. Pub. No 2004/0220471, entitled "Method and Device for Transseptal Facilitation Using Location System," published November 4, 2004, now abandoned; and U.S. Pub. No 2004/0220461, entitled "Transseptal Facilitation Using Sheath with Electrode Arrangement," published November 4, 2004, now abandoned. Some transseptal needle instruments include a dilation feature that further expands the opening formed through the atrial septum.

While transeptal puncture systems and methods have been made and used, improvements to the fluid communication functionality, user workflow, and other improvements are desired. It is believed that no one prior to the inventors has made or used the invention described, illustrated, and claimed herein.

In WO2022046777A1, there is described a self-contained, battery powered transseptal crossing system.

In WO2022001670A1, there is described a puncture needle and a puncture system. The puncture needle comprises an electrode tip and a sleeve sleeved on the electrode tip. The electrode tip is partially accommodated in the sleeve, so that the distal end of the electrode tip is exposed out of the sleeve; a fluid passage is formed between the electrode tip and the sleeve.

In US2022032007A1, there is described devices and methods for controlling the position of a deployable catheter instrument. In one embodiment, a catheter can include an instrument slidably disposed within an inner lumen of the catheter and coupled to at least one protrusion, as well as a retraction stop coupled to the catheter.

In US2016374751A1, there is described a method and apparatus for providing forward fluid delivery through an electrosurgical device, while avoiding coring when energy is delivered to the electrosurgical device.

In US2010168728A1, there is described an irrigated ablation catheter including a tubular catheter body having an inner tubular member and an outer tubular member.

### BRIEF DESCRIPTION OF THE DRAWINGS

The drawings and detailed description that follow are intended to be merely illustrative and are not intended to limit the scope of the invention as contemplated by the inventors.
FIG. 1 depicts a schematic view of a medical procedure in which a catheter of a catheter assembly is inserted in a patient;
FIG. 2 depicts a perspective view of an example of a guiding sheath that may be used with the catheter assembly of FIG. 1;
FIG. 3A depicts a schematic view of an example of a sheath approaching an atrial septum;
FIG. 3B depicts a schematic view of a dilator advanced distally relative to the sheath of FIG. 3A, such that the dilator is approaching the atrial septum;
FIG. 3C depicts a schematic view of a needle instrument advanced distally relative to the sheath of FIG. 3A and the dilator of FIG. 3B, such that the needle is penetrating the atrial septum to form an opening;
FIG. 3D depicts a schematic view of the dilator of FIG. 3B advanced distally through the opening formed by the needle instrument of FIG. 3C, such that the dilator is dilating the opening formed by the needle;
FIG. 3E depicts a schematic view of the dilator of FIG. 3B and the sheath of FIG. 3A retracted proximally, thereby exiting the dilated opening in the atrial septum;
FIG. 4 depicts a distal end view of the assembly formed by the needle instrument of FIG. 3C, the dilator of FIG. 3B, and the sheath of FIG. 3A;
FIG. 5 depicts a side elevational view of the needle instrument of FIG. 3C, with an intermediate portion of the needle instrument omitted;
FIG. 6 depicts a side elevational view of a distal portion of the needle instrument of FIG. 3C;
FIG. 7 depicts a side cross-sectional view of the distal portion of the needle instrument of FIG. 3C;
FIG. 8 depicts an elevation view of a distal portion of an example of a needle instrument, with a shaft of the needle instrument shown in lateral cross-section;
FIG. 9 depicts an end view of the distal end of the needle instrument of FIG. 8;
FIG. 10 depicts a cross-sectional view of the needle instrument of FIG. 8, taken along line 6-6 of FIG. 8;
FIG. 11 depicts an elevation view of a distal portion of another example of a needle instrument, with a shaft of the needle instrument shown in lateral cross-section;
FIG. 12 depicts an end view of the distal end of the needle instrument of FIG. 11;
FIG. 13 depicts a cross-sectional view of the needle instrument of FIG. 11, taken along line 9-9 of FIG. 11;
FIG. 14 depicts an elevation view of a distal portion of another example of a needle instrument;
FIG. 15 depicts a lateral cross-sectional view of the needle instrument of FIG. 14;
FIG. 16A depicts a lateral cross-sectional view of an example of a handle assembly for the needle instrument of FIG. 8, with a depth stop member in a proximal position and the handle assembly in a proximal position such that the handle assembly is spaced away from the guiding sheath of FIG. 2;
FIG. 16B depicts a lateral cross-sectional view of the handle assembly of FIG. 16A, with a depth stop member in a distal position and the handle assembly in the proximal position such that the handle assembly remains spaced away from the guiding sheath of FIG. 2; and
FIG. 16C depicts a lateral cross-sectional view of the handle assembly of FIG. 16A, with a depth stop member in a distal position and the handle assembly in a distal position such that the depth stop member engages the guiding sheath of FIG. 2.

### DETAILED DESCRIPTION FOR MODES OF CARRYING OUT THE INVENTION

The invention is defined by the appended claims.

The following description of certain examples of the invention should not be used to limit the scope of the present invention. The drawings, which are not necessarily to scale, depict selected embodiments and are not intended to limit the scope of the invention. The detailed description illustrates by way of example, not by way of limitation, the principles of the invention. Other examples, features, aspects, embodiments, and advantages of the invention will become apparent to those skilled in the art from the following description, which is by way of illustration, one of the best modes contemplated for carrying out the invention. As will be realized, the invention is capable of other different or equivalent aspects, all without departing from the invention. Accordingly, the drawings and descriptions should be regarded as illustrative in nature and not restrictive.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g., "about 90%" may refer to the range of values from 70% to 110%. In addition, as used herein, the terms "patient," "host," "user," and "subject" refer to any human or animal subject and are not intended to limit the systems or methods to human use, although use of the subject invention in a human patient represents a preferred embodiment.

### I. Overview of Example of a Catheter System

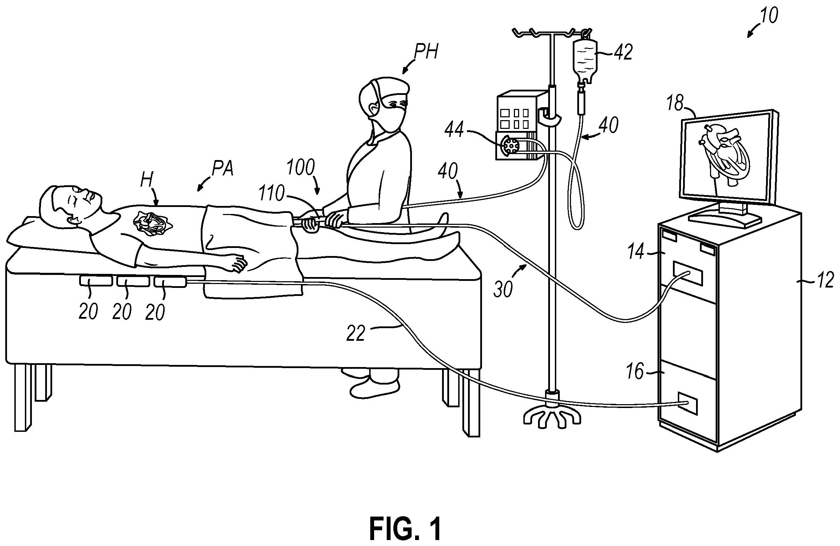

FIG. 1 shows an exemplary medical procedure and associated components of a cardiac catheter system that may be used to provide EP mapping or cardiac ablation as referred to above. In particular, FIG. 1 shows a physician (PH) grasping a handle assembly (110) of a catheter assembly (100), with an end effector (not shown) of a catheter of catheter assembly (100) disposed in a patient (PA) to map potentials in tissue and/or ablate tissue in or near the heart (H) of the patient (PA). The end effector may include various electrodes, sensors, and/or other features that are configured to deliver electrical energy to targeted tissue sites, provide EP mapping functionality, track external forces imparted on the end effector, track the location of end effector, and/or disperse fluid.

Catheter assembly (100) is coupled with a guidance and drive system (10) via a cable (30). Catheter assembly (100) is also coupled with a fluid source (42) via a fluid conduit (40). A set of field generators (20) are positioned underneath the patient (PA) and are coupled with guidance and drive system (10) via another cable (22). Magnetic field generators (20) are merely optional. Guidance and drive system (10) of the present example include a console (12) and a display (18). Console (12) includes a first driver module (14) and a second driver module (16). First driver module (14) is coupled with catheter assembly (100) via cable (30). In some variations, first driver module (14) is operable to receive EP mapping signals obtained via microelectrodes of the end effector of catheter assembly (100). Console (12) includes a processor (not shown) that processes such EP mapping signals and thereby provides EP mapping as is known in the art.

First driver module (14) of the present example is further operable to provide electrical power to a distal tip member of the end effector of catheter assembly (100), to thereby ablate tissue. Second driver module (16) is coupled with magnetic field generators (20) via cable (22). Second driver module (16) is operable to activate field generators (20) to generate an alternating magnetic field around the heart (H) of the patient (PA). For instance, field generators (20) may include coils that generate alternating magnetic fields in a predetermined working volume that contains the heart (H).

First driver module (14) is also operable to receive position indicative signals from a navigation sensor assembly in or near the end effector of catheter assembly (100). In such versions, the processor of console (12) is also operable to process the position indicative signals from the navigation sensor assembly to thereby determine the position of the end effector of catheter assembly (100) within the patient (PA). In some versions, the navigation sensor assembly includes two or more coils that are operable to generate signals that are indicative of the position and orientation of the end effector of catheter assembly (100) within the patient (PA). The coils are configured to generate electrical signals in response to the presence of an alternating electromagnetic field generated by magnetic field generators (20).

Display (18) is coupled with the processor of console (12) and is operable to render images of patient anatomy. Such images may be based on a set of preoperatively or intraoperatively obtained images (e.g., a CT or MRI scan, 3-D map, etc.). The views of patient anatomy provided through display (18) may also change dynamically based on signals from the navigation sensor assembly of the end effector of catheter assembly (100). For instance, as the end effector of catheter assembly (100) moves within the patient (PA), the corresponding position data from the navigation sensor assembly may cause the processor of console (12) to update the patient anatomy views in display (18) in real time to depict the regions of patient anatomy around the end effector as the end effector moves within the patient (PA). Moreover, the processor of console (12) may drive display (18) to show locations of aberrant conductive tissue sites, as detected via electrophysiological (EP) mapping with the end effector of catheter assembly (100) or as otherwise detected (e.g., using a dedicated EP mapping catheter, etc.). By way of example only, the processor of console (12) may drive display (18) to superimpose the locations of aberrant conductive tissue sites on the images of the patient's anatomy, such as by superimposing an illuminated dot, a crosshair, or some other form of visual indication of aberrant conductive tissue sites.

The processor of console (12) may also drive display (18) to superimpose the current location of the end effector of catheter assembly (100) on the images of the patient's anatomy, such as by superimposing an illuminated dot, a crosshair, a graphical representation of the end effector, or some other form of visual indication. Such a superimposed visual indication may also move within the images of the patient anatomy on display (18) in real time as the physician moves the end effector of catheter assembly (100) within the patient (PA), thereby providing real-time visual feedback to the operator about the position of the end effector within the patient (PA) as the end effector moves within the patient (PA). The images provided through display (18) may thus effectively provide a video tracking the position of the end effector of catheter assembly (100) within a patient (PA), without necessarily having any optical instrumentation (i.e., cameras) viewing the end effector. In the same view, display (18) may simultaneously visually indicate the locations of aberrant conductive tissue sites detected through EP mapping. The physician (PH) may thus view display (18) to observe the real time positioning of the end effector of catheter assembly (100) in relation to the mapped aberrant conductive tissue sites and in relation to images of the adjacent anatomical structures in the patient (PA).

Fluid source (42) of the present example includes a bag containing saline or some other suitable fluid. Conduit (40) includes a flexible tube that is further coupled with a pump (44), which is operable to selectively drive fluid from fluid source (42) to catheter assembly (100). Such fluid may be expelled through openings of the distal tip member of the end effector of catheter assembly (100). Such fluid may be provided in any suitable fashion as will be apparent to those skilled in the art in view of the teachings herein.

### II. Example of Guiding Sheath

In some procedures, the physician (PH) may wish to introduce a catheter of catheter assembly (100) into the patient (PA) via a guiding sheath. In some such procedures, the guiding sheath may be inserted into the patient (PA) (e.g., via the leg or groin of the patient (PA)); and then be advanced along a vein or artery to reach a position in or near the heart (H). Once the guiding sheath is suitably positioned in the patient (PA), the physician (PA) may then advance the end effector and catheter of catheter assembly (100) into the guiding sheath until the end effector exits the distal end of the guiding sheath. The physician (PA) may then operate catheter assembly (100) to provide EP mapping, ablation, or any other kind of operations in or near the heart (H) of the patient (PA).

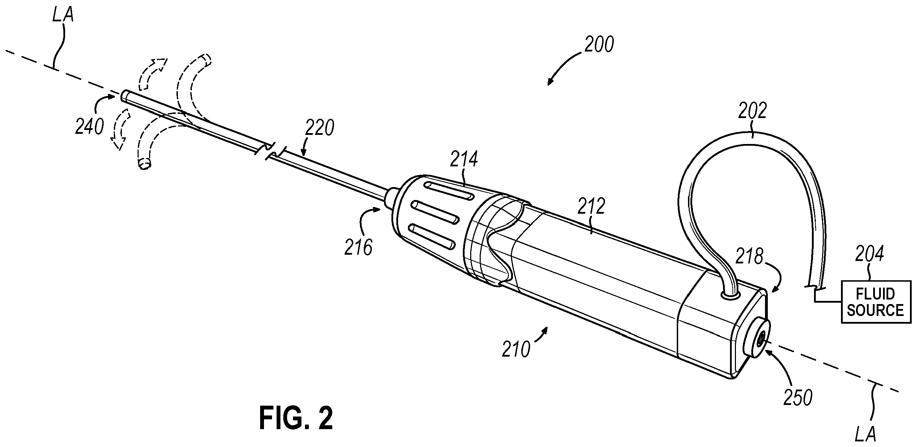

FIG. 2 shows an example of a guiding sheath (200) that may be used in such procedures. Guiding sheath (200) of this example includes a body in the form of a handle assembly (210) with a hollow shaft (220) extending distally from a distal end (216) of handle assembly (210). Handle assembly (210) is configured for grasping by a casing (212). The open distal end (240) of the hollow shaft (220) is operable to deflect laterally away from a longitudinal axis (LA) of the shaft. This deflection is controlled by a rotary knob (214) at distal end (216) of handle assembly (210). Rotary knob (214) is rotatable relative to casing (212), about the longitudinal axis (LA), to thereby actuate components that drive lateral deflection of open distal end (240) of hollow shaft (220). By way of example only, such actuation components may include one or more pull wires, bands, or any other suitable structures as will be apparent to those skilled in the art in view of the teachings herein. While the body of guiding sheath (200) is in the form of handle assembly (210) in this example, the body may take other forms. For instance, some variations may provide a robotically controlled form of guiding sheath (200). In some such variations, the body may take the form of a structure that mechanically interfaces with a robotic arm and/or other kind of robotic driving feature.

As shown in FIG. 2, a tube (202) extends laterally from the proximal end (218) of handle assembly (210). Tube (202) of this example is in fluid communication with a hollow interior (not shown) defined within handle assembly (210), with the hollow interior being in fluid communication with the interior of hollow shaft (220). Tube (202) of the present example is further in fluid communication with a fluid source (204). By way of example only, fluid source (204) may contain saline, a fluoroscopy contrast fluid, or any other suitable fluid. In some variations, fluid is communicated via an instrument that is disposed within hollow shaft (220), such as will be described in greater detail below in the context of needle instrument (290). In some such versions, tube (202) is omitted; and the instrument within hollow shaft (220) is directly coupled with fluid source (204).

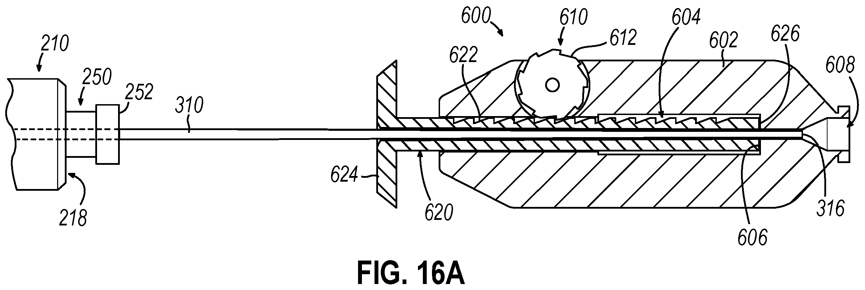

As also shown in FIG. 2, proximal end (218) of handle assembly (210) further includes an insertion port (250). Insertion port (250) is aligned with the longitudinal axis (LA) and provides a port for inserting the end effector and catheter of catheter assembly (100) into hollow shaft (220). Insertion port (250) of this example includes an annular protrusion (252) (e.g., as shown in FIG. 16A) defining an opening. Protrusion (252) protrudes proximally from casing (212) at proximal end (218). In some versions, protrusion (252) is omitted.

A seal (not shown) is positioned within the opening of insertion port (250). By way of example only, the seal may include an elastomeric membrane or other kind of component(s) as will be apparent to those skilled in the art in view of the teachings herein. The seal in the opening of insertion port (250) may further include a slit arrangement that is configured to facilitate insertion of an instrument (e.g., catheter of catheter assembly (100), etc.) through the seal. When nothing is inserted through the seal in the opening of insertion port (250), the seal is configured to provide a fluid-tight seal that prevents fluid from escaping the portion of the above-described fluid path defined within handle assembly (210) via insertion port (250); and prevents air from entering the above-described fluid path defined within handle assembly (210) via insertion port (250). When an instrument is inserted through the seal in the opening of insertion port (250), the seal still substantially maintains a fluid-tight seal of insertion port (250), preventing fluid from escaping the above-described fluid path defined within handle assembly (210) via insertion port (250); and preventing air from entering above-described fluid path defined within handle assembly (210) via insertion port (250), while still allowing the inserted instrument to translate relative to the seal in the opening of insertion port (250). Thus, regardless of whether an instrument is disposed in insertion port (250), the seal may prevent fluids from leaking out through insertion port (250) and prevent air from being aspirated into the heart (H) of the patient (PA) via insertion port (250).

### III. Example of Transseptal Penetration in Heart

### A. Overview

In some procedures, it may be desirable to have the end effector of catheter assembly (100) traverse the atrial septum in order to reach a targeted cardiovascular structure. For instance, the end effector may enter the heart (H) via the right atrium; while the targeted cardiovascular structure is a pulmonary vein, which is extends from the left atrium. With the atrial septum presenting an anatomical barrier between the right atrium and the left atrium, a perforating instrument may be used to form an opening through the atrial septum to thereby provide a passageway for the end effector of catheter assembly (100) to reach the left atrium and adjacent structures from the right atrium.

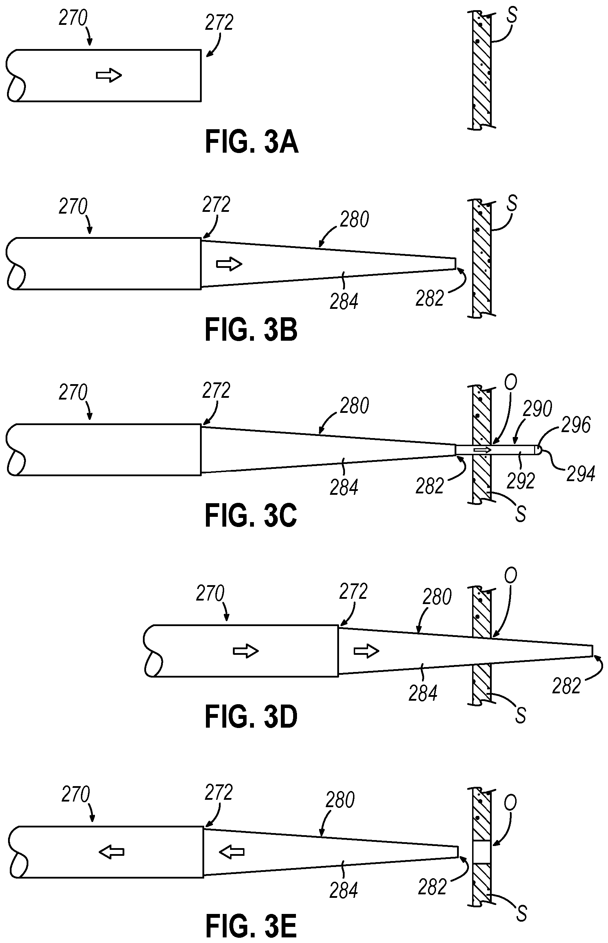

FIGS. 3A-3E show an example of a procedure where a needle instrument (290) is used to form an opening (O) through an atrial septum (S); and a dilator (280) is used to enlarge the opening (O). As shown in FIG. 3A, the procedure begins with a sheath (270) that is positioned within the right atrium, such that an open distal end (272) of sheath (270) is oriented toward a penetration target location of the septum (S). By way of example only, sheath (270) may include a universal guiding sheath such as guiding sheath (200) described above; or may be a sheath that is configured specifically for use with dilator (280) and needle instrument (290) (e.g., as part of a kit, etc.).

Once distal end (272) of sheath (270) has been appropriately positioned, dilator (280) is advanced distally relative to sheath (270), such that dilator (280) protrudes distally from distal end (272) of sheath (270) as shown in FIG. 3B. Dilator (280) of this example has an open distal end (282) and a tapered outer surface (284) that narrows toward distal end (282). In some versions, tapered outer surface (284) has a linear taper. In some other versions, tapered outer surface (284) has a curved taper. Alternatively, tapered outer surface (284) may have any other suitable configuration. With dilator (280) advanced distally relative to sheath (270), distal end (282) of dilator (280) is oriented toward the penetration target location of the septum (S).

Once distal end (282) of dilator (280) has been appropriately positioned, needle instrument (290) is advanced distally relative to dilator (280), such that a distal shaft (292) of needle instrument (290) protrudes distally from distal end (282) of dilator (280) as shown in FIG. 3C. As needle instrument (290) is advanced distally relative to dilator (280), a distal tip (294) and a portion of distal shaft (262) of needle instrument (290) penetrate the septum (S), thereby forming an opening (O). In the present example, distal tip (294) has a blunt configuration. For instance, distal tip (294) may have a dome shape or any other suitable configuration. Thus, while the term "needle" is used in the term needle instrument (290), this should not be read as requiring needle instrument (290) to have a sharp distal tip (294) (though some versions of needle instrument (290) may in fact have a sharp distal tip (294)). Distal tip (294) of the present example further includes an electrode (296), which is activated to apply electrical energy (e.g., radiofrequency (AC type) or pulsed field (DC type) energy) to the tissue of the septum (S), to thereby assist in penetration of the septum (S). While one electrode (296) is shown, distal tip (294) may alternatively include two or more electrodes (296).

After needle instrument (290) has formed the opening (O), dilator (280) is advanced distally into the opening (O) as shown in FIG. 3D. In the example shown in FIG. 3D, needle instrument (290) is retracted proximally back into dilator (280) before dilator (280) is advanced distally into the opening (O). In some other scenarios, needle instrument (290) remains distally advanced relative to dilator (280) when dilator (280) is advanced distally into the opening (O). As also shown in FIG. 3D, sheath (270) is advanced distally with dilator (280) when dilator (280) is advanced distally into the opening (O). In some other scenarios, sheath (270) remains stationary while dilator (280) is advanced distally into the opening (O). Regardless of the relative positioning of sheath (270) and needle instrument (290) when dilator (280) is advanced distally into the opening (O), tapered outer surface (284) of dilator (280) bears against the inner edge of the opening (O), thereby dilating the opening (O).

Once dilator (280) has sufficiently dilated the opening (O), dilator (280) is retracted proximally relative to the opening (O), such that dilator (280) exits the dilated opening (O), as shown in FIG. 3E. In the example shown, sheath (270) is retracted proximally with dilator (280). In some scenarios, dilator (280) and needle instrument (290) are completely removed from sheath (270) after reaching the state shown in FIG. 3E, while sheath (270) remains disposed in the right atrium. Then, an additional instrument (e.g., cardiac mapping catheter, cardiac ablation catheter, etc.) may be inserted through sheath (270) and then through the dilated opening (O). In some other scenarios, sheath (270) is removed from the right atrium and another sheath (e.g., guiding sheath (200)) is introduced into the right atrium; with additional instrumentation (e.g., cardiac mapping catheter, cardiac ablation catheter, etc.) being advanced through that other sheath to ultimately pass through the dilated opening (O).

By way of example only, the end effector of catheter assembly (100) may be advanced from the right atrium through the dilated opening (O) to thereby position the end effector of catheter assembly (100) in the left atrium, in a pulmonary vein, and/or in any other suitable anatomical region on that side of the septum (S). In some scenarios, sheath (270), or hollow shaft (220) of guiding sheath (200), is passed through the dilated opening (O) to further assist in guiding the end effector of catheter assembly (100) into the left atrium, into a pulmonary vein, and/or into any other suitable anatomical region on that side of the septum (S). At the completion of the procedure, the opening (O) may be closed using any suitable techniques.

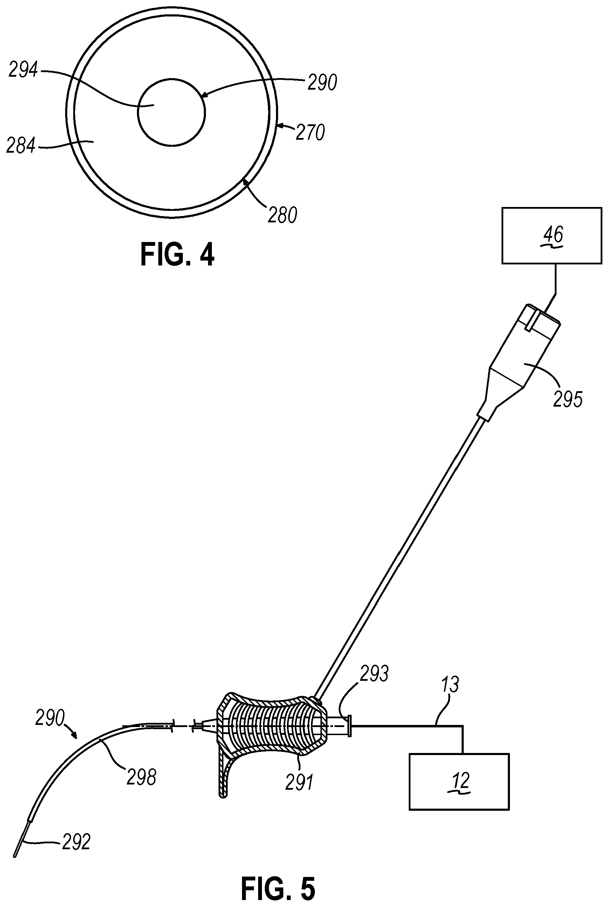

FIG. 4 provides a distal end view of the combination of sheath (270), dilator (280), and needle instrument (290). In the present example, sheath (270) and dilator (280) are configured such that the outer diameter of dilator (280) closely corresponds with outer diameter of sheath (270) when dilator (280) is distally advanced relative to sheath (270) as shown in FIG. 3B. In other words, there is a smooth transition between the outer diameter of dilator (280) and the outer diameter of sheath (270) at distal end (272) of sheath (270). This may reduce a risk of snagging at the transition between the outer diameter of dilator (280) and the outer diameter of sheath (270) at distal end (272) of sheath (270). In some versions, distal end (272) is beveled, rounded, or otherwise configured to provide a smooth transition from the outer diameter of sheath (270) to the outer diameter of dilator (280).

FIG. 5 shows needle instrument (290) in further detail. As shown, distal shaft (292) is coupled with a proximal shaft (298), which extends distally from a grip (291). Grip (291) further includes a proximal port (293) and a fluid coupling (295). Proximal port (293) is coupled with console (12) via a cable (13), which may contain one or more wires, flex circuit components, and/or other electrical communication components. Proximal port (293) thus provides an interface for electrical communication between cable (295) and one or more wires, traces, or other electrical communication components within needle instrument (290) that ultimately lead to electrode (296), navigation sensor(s), and/or any other kind(s) of electrical components in needle instrument (290). In some versions, console (12) is operable to provide electrical energy to needle assembly (290), catheter assembly (100), and/or other instrumentation. In some other versions, needle instrument (290) is coupled with some other electrical source (e.g., an electrical source dedicated to needle instrument (290)). Fluid coupling (295) of the present example is configured to couple with a fluid source (46). Fluid source (46) may contain saline, a fluoroscopy contrast fluid, and/or any other suitable kind of fluid. Fluid from fluid source (46) may reach needle instrument (290) via fluid coupling (295) and may ultimately be expelled from needle instrument (290) in accordance with the teachings provided below.

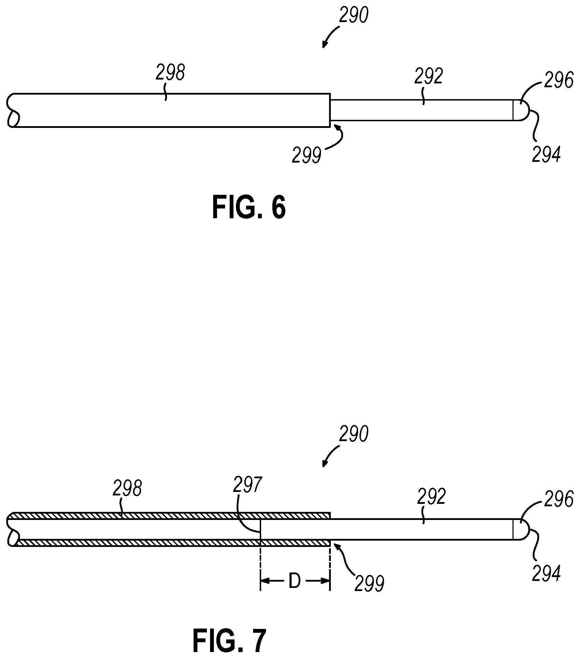

FIGS. 6-7 show a distal portion of needle assembly (290) in greater detail. As shown, a proximal portion of distal shaft (292) is inserted into proximal shaft (298), such that a proximal end (297) of distal shaft (292) is proximally positioned relative to distal end (299) of proximal shaft (299) by a distance (D). By way of example only, this distance (D) may be approximately 0.5 inches. Alternatively, distance (D) may have any other suitable value. In the present example, distal shaft (292) is fixedly secured to proximal shaft (298) via welding. Alternatively, distal shaft (292) may be fixedly secured to proximal shaft (298) via adhesive and/or in any other suitable fashion. As can be seen in FIGS. 6-7, there is a stepped transition at distal end (299), between the outer diameter of proximal shaft (298) and the outer diameter of distal shaft (292). In some versions, this configuration at distal end (299) engages an internal surface near distal end (282) of dilator (280) when needle assembly (290) is advanced distally relative to dilator (280) as shown in the sequence from FIG. 3B to FIG. 3C. In other words, the configuration at distal end (299) and the internal surface near distal end (282) may cooperate to provide a depth stop that restricts the distance to which needle assembly (290) may be advanced distally relative to dilator (280). Alternatively, any other suitable structure(s) may be used to restrict the distance to which needle assembly (290) may be advanced distally relative to dilator (280).

In addition to the foregoing, or in the alternative to the foregoing, needle instrument (290) may be configured and operable in accordance with at least some of the teachings of U.S. Pat. No 6,994,094; U.S. Pat. No 8,152,829; U.S. Pat. No 9,848,943; U.S. Pub. No 2004/0220471; and/or U.S. Pub. No 2004/0220461. Some dilators (280) and/or needle instruments (290) may also include one or more pull-wires and/or other features that provide steerability to the distal end of dilator (280) and/or needle instrument (290). Some such versions may thus provide an additional degree of steerability after distal end (280) of dilator (280) and/or distal tip (2 of the instrument exits open distal end (240) of hollow shaft (220). Some transseptal needle instruments may also include a wire at tip (264), to assist in providing primary engagement and lead-in for tip (264) into the atrial septum (S).

It is noted that a transseptal puncture apparatus outfitted with an energized tip, while described in specific reference to needle examples herein, may alternatively take the form of a guidewire. The differences in construction between a guidewire or needle will pertain primarily to the materials and construction of the shaft, with the choice between guidewire versus needle for transseptal access being primarily a function of physician preference. Accordingly, as used herein a "transseptal puncture apparatus" or "transseptal apparatus" means either a guide wire or needle having the tip features and/or depth stop features described herein within the scope of appended claims.

### A. Examples of Fluid Communication Features for Transseptal Needle Assemblies

In some versions of needle assembly (290) that are operable to apply electrical energy to tissue, it may be desirable to further enable such versions to provide a fluid (e.g., contrast or saline) at tip (264). Such fluid may provide contrast for use under fluoroscopy, saline for intracardiac echocardiogram (ICE), pressure monitor, or other local application of fluids as may be desired.

### 1. Example of Transseptal Needle Assembly with Discrete Recesses in Tip

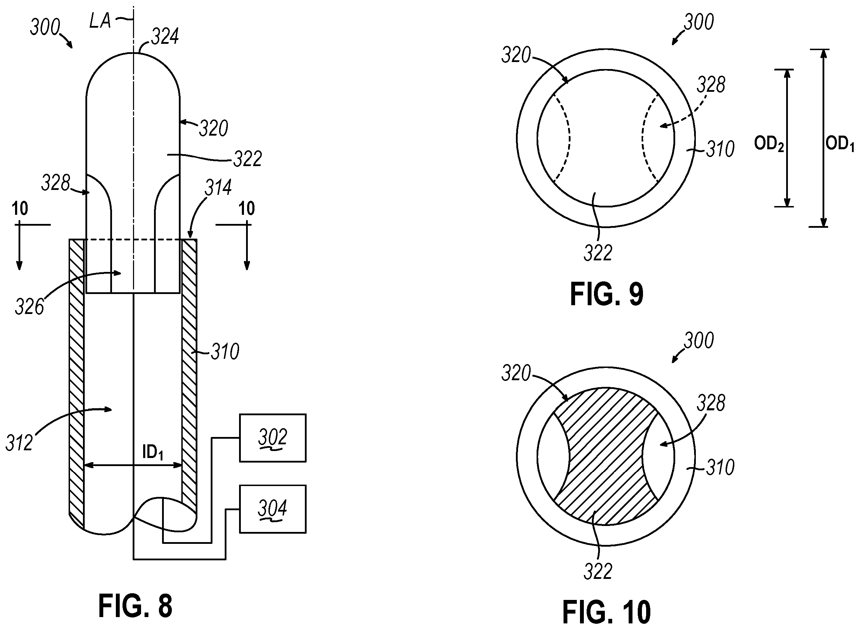

FIGS. 8-10 show a distal portion of a needle assembly (300) that may be used to form an opening (O) through an atrial septum (S) as described above with reference to FIGS. 3A-3E. In other words, needle assembly (300) may be incorporated into needle instrument (290). Needle assembly (300) of this example includes a shaft (310) and a tip member (320). Shaft (310) defines a lumen (312) that is fluidically coupled with a fluid source (302). Fluid source (302) may include a saline bag, a pump, syringe, and/or any other suitable components. Shaft (310) is sized for insertion through dilator (280). Sheath (270) and dilator (280) may thus be used to assist in positioning tip member (320) in relation to a penetration target location of the septum (S) as described above with reference to FIGS. 3A-3E. Shaft (310) may comprise a metallic material (e.g., stainless steel, such as 316SS), a plastic material, and/or any other suitable kind(s) of material(s) or combinations thereof. For example, in some examples the shaft may be composed of nylon and a polyether block amide (such as Pebax^{®}) combined with a stainless steel braiding. The exterior surface of shaft (310) also may be covered with an electrically insulative coating (not shown), such as Polytetrafluoroethylene (PTFE).

In some versions, shaft (310) and/or tip member (320) includes one or more navigation sensors also referred to as position or location sensors (not shown) that are operable to generate signals (e.g., in response to the presence of an alternating electromagnetic field generated by magnetic field generators (20)) that are indicative of the position and orientation of shaft (310) within the patient (PA). The navigation sensor(s) may be disposed on the shaft (310) in a predefined spatial relation to the tip member (320). The sensor(s) may include a magnetic sensor and/or at least one shaft electrode. The magnetic sensor may include at least one coil, for example, but not limited to, a dual-axis or a triple axis coil arrangement to provide position data for location and orientation including roll. The system (10) may determine a position and orientation of the shaft (310) and/or tip member (320) of needle assembly (300) based on signals provided by the magnetic sensor and/or the shaft electrodes fitted on shaft (310), e.g., on either side of the magnetic sensor.

Tip member (320) of the present example includes a body (322) having a distal tip (324) and a proximal end (326). Distal tip (324) of the present example has a dome shape, such that distal tip (324) is atraumatic. In some other versions, distal tip (324) is sharp or has some other structural configuration. Proximal end (326) of tip member (320) is inserted into distal end (314) of shaft (310) and is fixedly secured to shaft (310). By way of example only, tip member (320) may be welded to shaft (310), soldered to shaft (310), adhered to shaft (310) using an adhesive or epoxy, press-fit into shaft (310), and/or fixedly secured to shaft (310) in any other suitable fashion. In some versions, tip member (320) includes one or more navigation sensors (not shown) that are operable to generate signals (e.g., in response to the presence of an alternating electromagnetic field generated by field generators (20)) that are indicative of the position and orientation of tip member (320) within the patient (PA).

Tip member (320) may be composed of any number of suitable materials suitable for conducting electrical (e.g., thermal) and preferably RF energy capable of destroying target tissue upon contact. In this example, tip member (320) is composed of platinum iridium (about 90% and 10% iridium), which is also beneficially radiopaque, allowing for increased visibility of the tip under fluoroscopy. Furthermore, tip member (320) may be manufactured via machining, plasma welding, or other means as will be appreciated in the art. Tip member (320) also may optionally include echogenic surface modifications, such as glass-blasting, dimpling, or other surface modifications known in the art to enhance the echogenicity of tip member (320) for the purpose of ultrasound imaging.

Body (322) of tip member (320) further includes a plurality of recesses (328) in this example. Recesses (328) function as port holes for fluid as described above. Recesses (328) in this example extend from proximal end (326) to longitudinally intermediate region of body (322). A proximal portion of each recess (328) is positioned proximally in relation to distal end (314) of shaft (310); while a distal portion of each recess (328) is positioned distally in relation to distal end (314) of shaft (310). Recesses (328) may extend to any suitable extent along the length of body (322). Similarly, recesses (328) may extend to any suitable depth within body (322). Recesses (328) are in fluid communication with lumen (312) of shaft (310); and with the environment outside of tip member (320). Thus, recesses (328) provide a pathway for fluid that is communicated through lumen (312) to exit distally out through distal end (314) of shaft (310). This enables the fluid to reach the region of tip member (320) protruding from distal end (314); and to reach the tissue of the septum (S). In some scenarios, recesses (328) may also provide a pathway for air or other gas to enter lumen (312) of shaft (310) via tip member (320), such that recesses (328) may form part of a ventilation path. In alternative examples, fewer (e.g., only one) or more recesses may be included as desired and may have any number of shapes and sizes, including circle(s), oval(s), rectangle(s), and the like.

As shown in FIGS. 8-9, shaft (310) has an outer diameter (OD₁) and an inner diameter (ID₁); while tip member (320) has an outer diameter (OD₂). The outer diameter (OD₂) of tip member (320) is approximately equal to the inner diameter (ID₁) of shaft (310); while being smaller than the outer diameter (OD₁) of shaft (310). In some variations, shaft (310) provides a taper at distal end (314), thereby providing a smooth transition from outer diameter (OD₂) to outer diameter (OD₁), which may reduce the force required to fully penetrate the septum (S) in versions where a distal portion of shaft (310) passes through the septum (S). In some versions, a stepped transition between outer diameter (OD₂) and outer diameter (OD₁) may serve as a depth stop restricting distal advancement of needle assembly (300) through the septum (S); or at least provide tactile feedback in the form of resistance when the physician (PH) has advanced needle assembly (300) through the septum (S) to a point where distal end (314) engages the septum (S). In some such scenarios, the physician (PH) may stop advancing needle assembly (300) once distal end (314) engages the septum (S). The length of the portion of tip member (320) that is distally exposed relative to shaft (310) may thus be at least slightly longer than the thickness of an atrial septum (S), to thereby provide full penetration of the atrial septum (S) by tip member (320). In scenarios where needle assembly (300) is used with dilator (280), stepped transition between outer diameter (OD₂) and outer diameter (OD₁) may serve as a depth stop restricting distal advancement of needle assembly (300) relative to dilator (280), as described above in the context of the stepped transition at distal end (299), between the outer diameter of proximal shaft (298) and the outer diameter of distal shaft (292) of needle instrument (290).

As noted, in some versions, tip member (320) is formed of a metallic material such as platinum iridium. In some such versions, recesses (328) are formed using a ball end mill. Alternatively, any other suitable manufacturing techniques may be used, including but not limited to any other suitable milling technique, metal injection molding, casting, etc. While tip member (320) has two recesses (328) in the present example, tip member (320) may have any other suitable number of recesses (328). For instance, some versions of tip member (320) have only one recess (328). Some other versions of tip member (320) have three recesses (328), four recesses (328), five recesses (328), six recesses (328), or more than six recesses (328). Recesses (328) are spaced apart from each other equidistantly about a central longitudinal axis (LA) extending through body (322) in this example, though any other suitable arrangement may be provided. Recess(es) (328) may likewise have any number of shapes and sizes, including circle(s), oval(s), slit(s), and the like.

As shown in FIG. 8, tip member (320) is also coupled with an electrical generator (304). For instance, one or more wires, traces, or other electrically conductive elements may extend along shaft (310) to electrically couple tip member (320) with electrical generator (304), which can generate RF energy (i.e., a high frequency AC signal) for thermal ablation or low voltage high energy DC current for non-thermal ablation (i.e., irreversible electroporation or pulsed field ablation). In versions employing a wire conductor between tip member (320) and generator (304), the wire may be composed of a nickel-cobalt base alloy, such as MP35N^{®} and joined to the tip via laser welding. Tip member (320) is operable to apply electrical energy to the tissue of the septum (S). Such electrical energy may assist tip member (320) in penetrating the septum (S). In some versions where tip member (320) is formed of a metallic material (e.g., platinum iridium as described above), the tip member (320) may serve as a single, monopolar electrical electrode, such that a ground pad may contact the patient (PA) to provide a return path for the electrical energy. In some such versions, particularly where shaft (310) is formed of an electrically conductive material, an electrically insulating material may be interposed between tip member (320) and shaft (310). Alternatively, tip member (320) may include an electrical electrode at distal tip (324), with an electrically insulating material being interposed between the electrode and body (322). As yet another variation, tip member (320) may have two or more electrical electrodes at distal tip (324), with such electrodes being operable to apply bipolar electrical energy to the tissue of the septum (S).

Tip member (320) may be composed of any number of suitable materials suitable for conducting electrical (e.g., thermal) and preferably RF energy capable of destroying target tissue upon contact. In this example, tip member (320) is composed of platinum iridium (about 90% and 10% iridium), which is also beneficially radiopaque, allowing for increased visibility of the tip under fluoroscopy. Furthermore, tip member (320) may be manufactured via machining, plasma welding, or other means as will be appreciated in the art. Tip member (320) also may optionally include echogenic surface modifications, such as glass-blasting, dimpling, or other surface modifications known in the art to enhance the echogenicity of tip member (320) for the purpose of ultrasound imaging.

### 2. Example of Transseptal Needle Assembly with Annular Gap in Tip

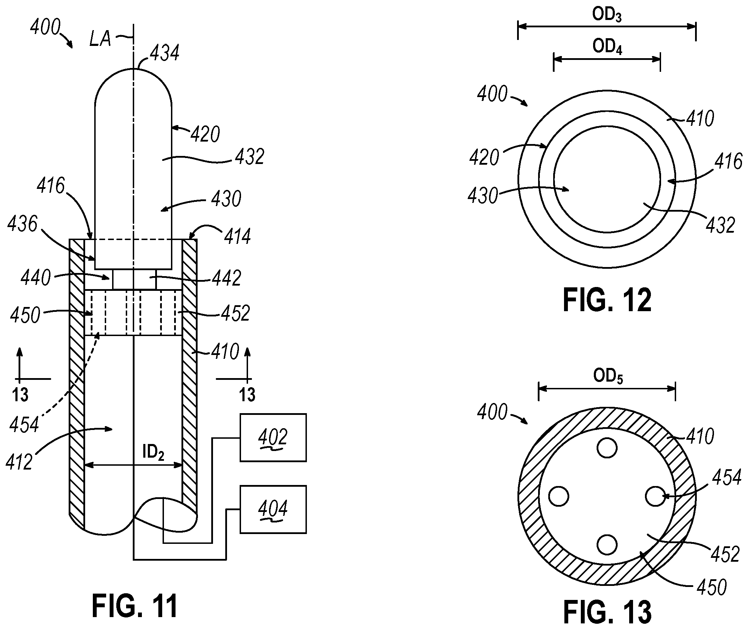

FIGS. 11-13 show a distal portion of another needle assembly (400) that may be used to form an opening (O) through an atrial septum (S) as described above with reference to FIGS. 3A-3E. In other words, needle assembly (400) may be incorporated into needle instrument (290). Needle assembly (400) of this example includes a shaft (410) and a tip member (420). Shaft (410) defines a lumen (412) that is fluidically coupled with a fluid source (402). Fluid source (402) may include a saline bag, a pump, syringe, and/or any other suitable components. Shaft (410) is sized for insertion through dilator (280). Sheath (270) and dilator (280) may thus be used to assist in positioning tip member (420) in relation to a penetration target location of the septum (S) as described above with reference to FIGS. 3A-3E. Shaft (410) may comprise a metallic material (e.g., stainless steel, such as 316SS), a plastic material, and/or any other suitable kind(s) of material(s) or combinations thereof. For example, in some examples the shaft may be composed of nylon and a polyether block amide (such as Pebax^{®}) combined with a stainless steel braiding. The exterior surface of shaft (410) also may be covered with an electrically insulative coating (not shown), such as Polytetrafluoroethylene (PTFE)

In some versions, shaft (410) and/or tip member (420) includes one or more navigation sensors (not shown) that are operable to generate signals (e.g., in response to the presence of an alternating electromagnetic field generated by field generators (20)) that are indicative of the position and orientation of shaft (410) within the patient (PA). The navigation sensor(s) may be disposed on the shaft (410) in a predefined spatial relation to the tip member (420). The sensor(s) may include a magnetic sensor and/or at least one shaft electrode. The magnetic sensor may include at least one coil, for example, but not limited to, a dual-axis or a triple axis coil arrangement to provide position data for location and orientation including roll. The system (10) may determine a position and orientation of the shaft (410) and/or tip member (420) of needle assembly (400) based on signals provided by the magnetic sensor and/or the shaft electrodes fitted on shaft (410), e.g., on either side of the magnetic sensor.

Tip member (420) of the present example includes a distal portion (430), an intermediate portion (440), and a proximal portion (450). Distal portion (430) includes a body (432) having a distal tip (434) and a proximal end (436). Distal tip (434) of the present example has a dome shape, such that distal tip (434) is atraumatic. In some other versions, distal tip (434) is sharp or has some other structural configuration. Proximal end (436) is integral with a body (442) of intermediate portion (440). Body (442) of intermediate portion (440) is also integral with a body (452) of proximal portion (450). Body (442) of the present example is narrower than bodies (432, 452), such that body (442) forms a narrowed neck region connecting body (432) to body (452). In some other versions, body (432) extends directly from body (452), such that body (442) may be omitted.

Proximal portion (450), intermediate portion (440), and proximal end (436) are positioned within distal end (414) of shaft (410). Tip member (420) is rigidly secured to shaft (410) via proximal portion (450). By way of example only, proximal portion (450) may be welded to shaft (410), soldered to shaft (410), adhered to shaft (410) using an adhesive or epoxy, press-fit into shaft (410), and/or fixedly secured to shaft (410) in any other suitable fashion. In some versions, tip member (420) includes one or more navigation sensors (not shown) that are operable to generate signals (e.g., in response to the presence of an alternating electromagnetic field generated by field generators (20)) that are indicative of the position and orientation of tip member (420) within the patient (PA).

Tip member (420) may be composed of any number of suitable materials suitable for conducting electrical (e.g., thermal) and preferably RF energy capable of destroying target tissue upon contact. In this example, tip member (420) is composed of platinum iridium (about 90% and 10% iridium), which is also beneficially radiopaque, allowing for increased visibility of the tip under fluoroscopy. Furthermore, tip member (420) may be manufactured via machining, plasma welding, or other means as will be appreciated in the art. Tip member (420) also may optionally include echogenic surface modifications, such as glass-blasting, dimpling, or other surface modifications known in the art to enhance the echogenicity of tip member (420) for the purpose of ultrasound imaging.

As shown in FIGS. 11-12, shaft (410) has an outer diameter (OD₃) and an inner diameter (ID₂); while distal portion (430) of tip member (420) has an outer diameter (OD₄). As shown in FIG. 13, body (452) of proximal portion (450) of tip member (420) has a disc shape and defines an outer diameter (OD₅). The outer diameter (OD₅) of proximal portion (450) is approximately equal to the inner diameter (ID₂) of shaft (410); while being smaller than the outer diameter (OD₃) of shaft (410). The outer diameter (OD₄) of distal portion (430) is smaller than the inner diameter (ID₂) of shaft (410), the outer diameter (OD₃) of shaft (410), and the outer diameter (OD₅) of proximal portion (450). Distal portion (430) thus defines a gap (416) between the exterior surface of body (432) and the inner surface of shaft (410). This gap (416) is in fluid communication with lumen (412); and with the environment outside of tip member (420). Body (452) of proximal portion (450) defines a plurality of fluid passageways (454) in the form of openings that are also in fluid communication with lumen (412). Thus, fluid passageways (454) and gap (416) together provide a pathway for fluid that is communicated through lumen (412) to exit distally out through distal end (414) of shaft (410). This enables the fluid to reach the region of tip member (420) protruding from distal end (414); and to reach the tissue of the septum (S). In some scenarios, fluid passageways (454) and gap (416) may also provide a pathway for air or other gas to enter lumen (412) of shaft (410), such that fluid passageways (454) and gap (416) may together form part of a ventilation path. While tip member (420) has four fluid passageways (454) in the present example, tip member (420) may have any other suitable number of fluid passageways (454). Fluid passageways (454) are spaced apart from each other equidistantly about a central longitudinal axis (LA) extending through body (452) in this example, though any other suitable arrangement may be provided.

In some versions, the stepped transition between the outer diameter (OD₄) of distal portion (430) and the outer diameter (OD₃) of shaft (410) may serve as a depth stop restricting distal advancement of needle assembly (400) through the septum (S); or at least provide tactile feedback in the form of resistance when the physician (PH) has advanced needle assembly (400) through the septum (S) to a point where distal end (414) engages the septum (S). In some such scenarios, the physician (PH) may stop advancing needle assembly (400) once distal end (414) engages the septum (S). The length of the portion of tip member (420) that is distally exposed relative to shaft (410) may thus be at least slightly longer than the thickness of an atrial septum (S), to thereby provide full penetration of the atrial septum (S) by tip member (420). In scenarios where needle assembly (400) is used with dilator (280), stepped transition between outer diameter (OD₄) and outer diameter (OD₃) may serve as a depth stop restricting distal advancement of needle assembly (400) relative to dilator (280), as described above in the context of the stepped transition at distal end (299), between the outer diameter of proximal shaft (298) and the outer diameter of distal shaft (292) of needle instrument (290).

As shown in FIG. 11, tip member (420) is also coupled with an electrical generator (404). For instance, one or more wires, traces, or other electrically conductive elements may extend along shaft (410) to electrically couple tip member (420) with electrical generator (404), which can generate RF energy (i.e., a high frequency AC signal) for thermal ablation or low voltage high energy DC current for non-thermal ablation (i.e., irreversible electroporation or pulsed field ablation). In versions employing a wire conductor between tip member (420) and generator (404), the wire may be composed of a nickel-cobalt base alloy, such as MP35N^{®} and joined to the tip via laser welding. Tip member (420) is operable to apply electrical energy to the tissue of the septum (S). Such electrical energy may assist tip member (420) in penetrating the septum (S). In some versions where tip member (420) is formed of a metallic material (e.g., platinum iridium), the tip member (420) may serve as a single, monopolar electrical electrode, such that a ground pad may contact the patient (PA) to provide a return path for the electrical energy. In some such versions, particularly where shaft (410) is formed of an electrically conductive material, an electrically insulating material may be interposed between tip member (420) and shaft (410). Alternatively, tip member (420) may include an electrical electrode at distal tip (434), with an electrically insulating material being interposed between the electrode and body (432). As yet another variation, tip member (420) may two or more electrical electrodes at distal tip (434), with such electrodes being operable to apply bipolar electrical energy to the tissue of the septum (S).

Tip member (420) may be composed of any number of suitable materials suitable for conducting electrical (e.g., thermal) and preferably RF energy capable of destroying target tissue upon contact. In this example, tip member (420) is composed of platinum iridium (about 90% and 10% iridium), which is also beneficially radiopaque, allowing for increased visibility of the tip under fluoroscopy. Furthermore, tip member (420) may be manufactured via machining, plasma welding, or other means as will be appreciated in the art. Tip member (420) also may optionally include echogenic surface modifications, such as glass-blasting, dimpling, or other surface modifications known in the art to enhance the echogenicity of tip member (420) for the purpose of ultrasound imaging.

### 3. Example of Transseptal Needle Assembly with Openings in Tip

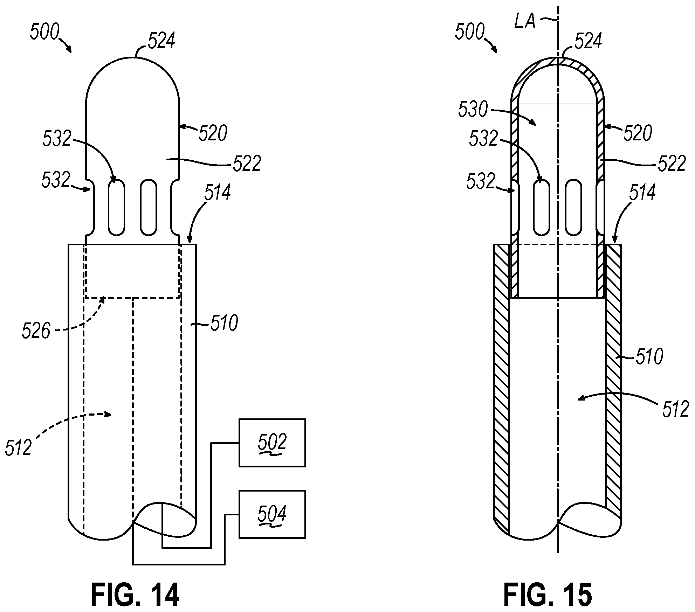

FIGS. 14-15 show a distal portion of another needle assembly (500) that may be used to form an opening (O) through an atrial septum (S) as described above with reference to FIGS. 3A-3E. In other words, needle assembly (500) may be incorporated into needle instrument (290). Needle assembly (500) of this example includes a shaft (510) and a tip member (520). Shaft (510) defines a lumen (512) that is fluidically coupled with a fluid source (502). Fluid source (502) may include a saline bag, a pump, syringe, and/or any other suitable components. Shaft (510) is sized for insertion through dilator (280). Sheath (270) and dilator (280) may thus be used to assist in positioning tip member (520) in relation to a penetration target location of the septum (S) as described above with reference to FIGS. 3A-3E. Shaft (510) may comprise a metallic material (e.g., stainless steel, such as 316SS), a plastic material, and/or any other suitable kind(s) of material(s) or combinations thereof. For example, in some examples the shaft may be composed of nylon and a polyether block amide (such as Pebax^{®}) combined with a stainless steel braiding. The exterior surface of shaft (310) also may be covered with an electrically insulative coating (not shown), such as Polytetrafluoroethylene (PTFE).

In some versions, shaft (510) and/or tip member (320) includes one or more navigation sensors (not shown) that are operable to generate signals (e.g., in response to the presence of an alternating electromagnetic field generated by field generators (20)) that are indicative of the position and orientation of shaft (510) within the patient (PA). The navigation sensor(s) may be disposed on the shaft (510) in a predefined spatial relation to the tip member (520). The sensor(s) may include a magnetic sensor and/or at least one shaft electrode. The magnetic sensor may include at least one coil, for example, but not limited to, a dual-axis or a triple axis coil arrangement to provide position data for location and orientation including roll. The system (10) may determine a position and orientation of the shaft (510) and/or tip member (520) of needle assembly (500) based on signals provided by the magnetic sensor and/or the shaft electrodes fitted on shaft (510), e.g., on either side of the magnetic sensor.

Tip member (520) of the present example includes a body (522) having a distal tip (524) and a proximal end (526). Distal tip (524) of the present example has a dome shape, such that distal tip (524) is atraumatic. In some other versions, distal tip (524) is sharp or has some other structural configuration. Proximal end (526) of tip member (520) is inserted into distal end (514) of shaft (510) and is fixedly secured to shaft (510). By way of example only, tip member (520) may be welded to shaft (510), soldered to shaft (510), adhered to shaft (510) using an adhesive or epoxy, press-fit into shaft (510), and/or fixedly secured to shaft (510) in any other suitable fashion. In some versions, tip member (520) includes one or more navigation sensors (not shown) that are operable to generate signals (e.g., in response to the presence of an alternating electromagnetic field generated by field generators (20)) that are indicative of the position and orientation of tip member (520) within the patient (PA).

Distal end (514) of shaft (510) may provide a stepped transition between the outer diameter of shaft (510) and the outer diameter of tip member (520). As described above, this stepped transition may provide a depth stop and/or tactile feedback when distal end (514) engages the septum (S). Alternatively, this stepped transition may provide a depth stop and/or tactile feedback when distal end (514) engages an internal structure near distal end (282) of dilator (280).

Tip member (520) may be composed of any number of suitable materials suitable for conducting electrical (e.g., thermal) and preferably RF energy capable of destroying target tissue upon contact. In this example, tip member (520) is composed of platinum iridium (about 90% and 10% iridium), which is also beneficially radiopaque, allowing for increased visibility of the tip under fluoroscopy. Furthermore, tip member (520) may be manufactured via machining, plasma welding, or other means as will be appreciated in the art. Tip member (520) also may optionally include echogenic surface modifications, such as glass-blasting, dimpling, or other surface modifications known in the art to enhance the echogenicity of tip member (520) for the purpose of ultrasound imaging.

Body (522) of tip member (520) is hollow, such that body (522) defines an interior space (530). Body (522) further includes a plurality of openings (532) in this example. While body (522) has six openings (532) in the present example, body (522) may have any other suitable number of openings (532). In the present example, openings (532) are in the form of elongate slots, though openings (532) may have any other suitable configuration(s), and may, for example, extend proximally to the proximate end (526) of the tip member so that one or more of the opening(s) takes the form of a slit opening. Similarly, openings (532) may have any other suitable arrangement on body (522) other than the arrangement shown in FIGS. 14-15. Openings (532) of the present example are in fluid communication with interior space (530); and with the environment outside of tip member (420). Interior space (530) is further in fluid communication with lumen (512) of shaft (510). Thus, openings (532) and interior space (530) together provide a pathway for fluid that is communicated through lumen (512) to exit distally out through tip member (520). This enables the fluid to cool body (522) of tip member (520); and to reach the tissue of the septum (S). In some scenarios, openings (532) and interior space (530) together may also provide a pathway for air or other gas to enter lumen (512) of shaft (510) via tip member (520), such that openings (532) and interior space (530) together may form part of a ventilation path.

As shown in FIG. 14, tip member (520) is also coupled with an electrical generator (504). For instance, one or more wires, traces, or other electrically conductive elements may extend along shaft (510) to electrically couple tip member (520) with electrical generator (504), which can generate RF energy (i.e., a high frequency AC signal) for thermal ablation or low voltage high energy DC current for non-thermal ablation (i.e., irreversible electroporation or pulsed field ablation). In versions employing a wire conductor between tip member (520) and generator (504), the wire may be composed of a nickel-cobalt base alloy, such as MP35N^{®} and joined to the tip via laser welding. Tip member (520) is operable to apply electrical energy to the tissue of the septum (S). Such electrical energy may assist tip member (520) in penetrating the septum (S). In some versions where tip member (520) is formed of a metallic material (e.g., platinum iridium as described above), the tip member (520) may serve as a single, monopolar electrical electrode, such that a ground pad may contact the patient (PA) to provide a return path for the electrical energy. In some such versions, particularly where shaft (510) is formed of an electrically conductive material, an electrically insulating material may be interposed between tip member (520) and shaft (510). Alternatively, tip member (520) may include an electrical electrode at distal tip (524), with an electrically insulating material being interposed between the electrode and body (522). As yet another variation, tip member (520) may have two or more electrical electrodes at distal tip (524), with such electrodes being operable to apply bipolar electrical energy to the tissue of the septum (S).

Tip member (520) may be composed of any number of suitable materials suitable for conducting electrical (e.g., thermal) and preferably RF energy capable of destroying target tissue upon contact. In this example, tip member (520) is composed of platinum iridium (about 90% and 10% iridium), which is also beneficially radiopaque, allowing for increased visibility of the tip under fluoroscopy. Furthermore, tip member (520) may be manufactured via machining, plasma welding, or other means as will be appreciated in the art. Tip member (520) also may optionally include echogenic surface modifications, such as glass-blasting, dimpling, or other surface modifications known in the art to enhance the echogenicity of tip member (520) for the purpose of ultrasound imaging.

### B. Example of Depth Stop Feature for Transseptal Needle Assembly

As described above, a guiding sheath (200) may be used to guide a needle instrument (290) or needle assembly (300, 400, 500) to a target penetration target location of an atrial septum (S). There may be scenarios where there is variation between the length of hollow shaft (220) of guiding sheath (200) and the lengths of the shafts (262) of different needle instruments (290). Similarly, there may be scenarios where there is variation between the length of hollow shaft (220) of guiding sheath (200) and the lengths of the shafts (310, 410, 510) of different needle assemblies (300, 400, 500). Such variations in respective lengths may require the physician (PH) to carefully determine how far needle assembly (300, 400, 500) should be distally advanced relative to guiding sheath (200), to avoid a scenario where needle instrument (290) or needle assembly (300, 400, 500) is inadvertently advanced too far to a point where distal tip (264, 324, 434, 524) engages (or even penetrates) a wall or other structure of the heart (H) that is past the atrial septum (S).

It may therefore be desirable to provide a depth limiting feature that may be adjusted before the procedure begins; where the depth limiting feature may mechanically restrict the depth to which needle instrument (290) or needle assembly (300, 400, 500) may be distally advanced relative to guiding sheath (200). Such a depth limiting feature may thus prevent inadvertent over-advancement of needle instrument (290) or needle assembly (300, 400, 500), and thereby prevent inadvertent engagement (or penetration) of distal tip (264, 324, 434, 524) with a wall or other structure of the heart (H) that is past the atrial septum (S).

FIGS. 16A-16C show an example of a depth limiting feature that may be adjusted and used to restrict the depth to which needle instrument (290) or needle assembly (300, 400, 500) may be distally advanced relative to guiding sheath (200). While guiding sheath (200) is shown and described in this example, the same teachings may be readily applied in the context where needle instrument (290) is inserted into sheath (270). In this example, the depth limiting feature is in the form of a depth stop member (620) that is incorporated into a handle assembly (600) of needle assembly (300). Handle assembly (600) may thus be substituted for grip (291) shown in FIG. 5. While handle assembly (600) is provided with needle assembly (300) in this example, handle assembly (600) may readily be provided with needle instrument (290), needle assemblies (400, 500), or any other suitable needle instrument/assembly.

Handle assembly (600) of this example includes a body (602) that defines a cavity (604) and a proximal port (608). Shaft (310) of needle assembly (300) is fixedly secured to body (602). By way of example only, shaft (310) may be fixedly secured to body (602) via overmolding, press-fitting, adhesive, and/or using any other suitable technique. Proximal end (316) is positioned adjacent to proximal port (608). In some versions, proximal port (608) provides a structure for coupling shaft (310) with a tube or other fluid communication feature. For instance, a tube that is coupled with proximal port (608) may provide a fluid pathway to a source of fluoroscopy contrast fluid. In some other versions, proximal port (608) provides a feature for coupling with a cable (13) (e.g., similar to port (293) of grip (291), etc.).

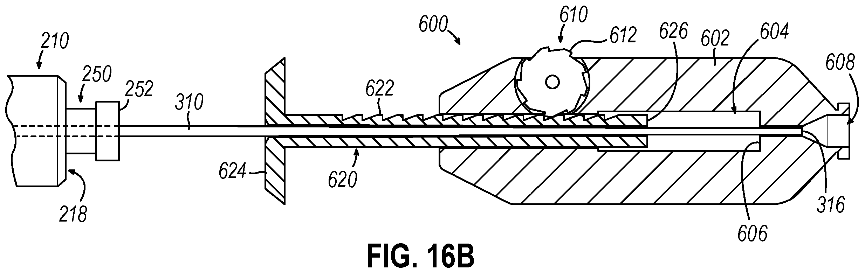

A thumbwheel (610) is rotatably supported by body (602) and includes a plurality of teeth (612). Depth stop member (620) is slidably coupled with body (602) and shaft (310), such that depth stop member (620) is operable to translate longitudinally relative to body (602) and shaft (310). Depth stop member (620) includes a set of teeth (622) that mesh with teeth (612) of thumbwheel (610), such that depth stop member (620) and thumbwheel (610) form a rack and pinion arrangement in this example. Thus, thumbwheel (610) may be rotated relative to body (620) to drive translation of depth stop member (620) between a first position (e.g., as shown in FIG. 16A) and a second position (e.g., as shown in FIG. 16B). Alternatively, any other suitable features may be used to drive translation of depth stop member (620) relative to body (602) and shaft (310), including but not limited to a slider, a pivoting rocker, a dial, etc.

Depth stop member (620) of the present example further includes a distal flange (624). Distal flange (624) is sized and configured to engage protrusion (252) of insertion port (250) of guiding sheath (200), to thereby arrest distal advancement of handle assembly (600) relative to guiding sheath (200). The longitudinal position of depth stop member (620) relative to body (602) and shaft (310) may thus be adjusted to thereby adjust the length to which shaft (310) and tip member (320) may protrude distally past distal end (240) of hollow shaft (220) of guiding sheath (200). In some versions, depth stop member (620) may include indicia that can be visually observed to identify an insertion depth, protrusion distance, or other value that might assist the physician (PH) in determining how far to advance depth stop member (620) relative to body (602) based on the respective lengths of shaft (310) and shaft (220) at hand. Similarly, depth stop member (620) may include detent features associated with predetermined length increments to assist the physician (PH) in appropriately gauging the adjusted position of depth stop member (620) relative to body (602). Such detent features may also substantially retain the relative longitudinal position of depth stop member (620) relative to body (602) once the desired longitudinal position has been reached. In addition, or in the alternative, handle assembly (600) may include a locking feature (e.g., a latch, etc.) that is operable to selectively lock or unlock the longitudinal position of depth stop member (620) relative to body (602).

In an example of use of handle assembly (600), depth stop member (620) may initially start in a proximal-most position as shown in FIG. 16A, where a proximal end (626) of depth stop member (620) is directly adjacent to a proximal end (606) of cavity (604). Then, the physician (PH) may rotate thumbwheel (610) to drive depth stop member (620) distally relative to body (602) and shaft (310), as shown in FIG. 16B, until depth stop member (620) reaches a desired longitudinal position relative to body (602) and shaft (310). In versions where handle assembly (600) includes a locking mechanism, the locking mechanism may be actuated at this stage to thereby lock the longitudinal position of depth stop member (620) relative to body (602) and shaft (310). While FIGS. 16A-16B show the depth stop member (620) adjustment process being carried out while shaft (310) is already disposed in guiding sheath (200), this adjustment process may alternatively be carried out before shaft (310) is inserted in guiding sheath (200). In scenarios where the adjustment process is carried out while a portion of shaft (310) is already disposed in guiding sheath (200), the adjustment process may nevertheless be carried out before shaft (310) is fully advanced in guiding sheath (200).

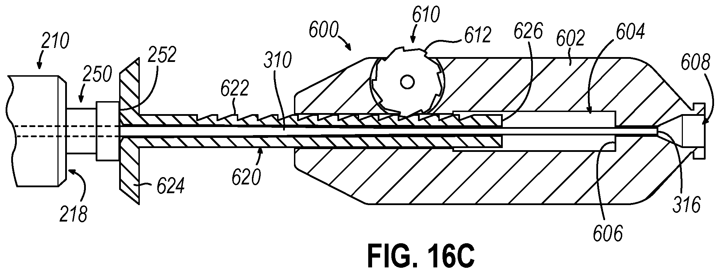

After depth stop member (620) is at the desired longitudinal position relative to body (602) and shaft (310), and shaft (310) is at least partially disposed in guiding sheath (200), the physician (PH) may then advance body (602) of handle assembly (600) distally toward guiding sheath (200) until distal flange (624) engages protrusion (252) of insertion port (250) as shown in FIG. 16C. As this stage, shaft (310) and tip member (320) protrude distally past distal end (240) of hollow shaft (220) of guiding sheath (200) to the extent governed by the longitudinal position of depth stop member (620) relative to body (602) and shaft (310). In some versions, the physician (PH) relies on visual feedback and/or tactile feedback indicating engagement between distal flange (624) of depth stop member (620) and protrusion (252) of insertion port (250) to determine that shaft (310) has been sufficiently advanced distally relative to guiding sheath (200); such that the physician (PH) stops such distal advancement upon seeing and/or feeling the engagement between distal flange (624) of depth stop member (620) and protrusion (252) of insertion port (250). In addition, or in the alternative, distal flange (624) of depth stop member (620) and protrusion (252) of insertion port (250) may cooperate to provide a hard stop that physically prevents any further distal advancement of shaft (310) relative to guiding sheath (200). In either scenario, depth stop member (620) may prevent, or assist in preventing, over-insertion of shaft (310) relative to guiding sheath (200).

As yet another variation, a clamping member may be selectively secured to a suitable location along the proximal region of shaft (262, 310, 410, 510) to serve as a depth stop. For instance, such a clamping member may engage protrusion (252) of insertion port (250), in a manner similar to that described above with respect to distal flange (624), to thereby arrest distal advancement of shaft (262, 310, 410, 510) relative to guiding sheath (200).

### V. Miscellaneous

Any of the instruments described herein may be cleaned and sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, hydrogen peroxide, peracetic acid, and vapor phase sterilization, either with or without a gas plasma, or steam.

Having shown and described various versions of the present invention, further adaptations of the methods and systems described herein may be accomplished by appropriate modifications by one skilled in the art without departing from the scope of the present invention. Several of such potential modifications have been mentioned, and others will be apparent to those skilled in the art. For instance, the examples, versions, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A transeptal apparatus, comprising:
(a) a body assembly;
(b) a shaft (410) extending distally from the body assembly, the shaft including:
(i) a distal end, and
(ii) a lumen (412); and
(c) at tip member (420) secured at the distal end of the shaft, the tip member and the distal end of the shaft being sized and configured to fit within a chamber of a heart of a human subject, the tip member including:
(i) a distal body (430) comprising:
a distal tip (434), the distal tip being configured to deliver electrical energy to tissue; and
a proximal end (436);
(ii) a proximal body (450) coupled with the distal body;
(iii) an intermediate body (440) interposed between the proximal body and the distal body;
(iv) at least one fluid passageway (454) in fluid communication with the lumen of the shaft, at least a portion of the at least one fluid passageway of the tip member being positioned proximally in relation to the distal end of the shaft;
the proximal body being secured to the shaft, the at least one fluid passageway being formed through the proximal body;
the lumen comprising an inner diameter (ID2), the distal body defining a first outer diameter (OD4), the proximal body defining a second outer diameter (OD5), the second outer diameter being sized to correspond with the inner diameter, the first outer diameter being smaller than the inner diameter such that the distal body defines a gap (416) with the shaft, the gap and the at least one fluid passageway formed through the proximal body being configured to cooperate to provide a pathway for fluid communication between the lumen and a region exterior to the tip member;
the intermediate body being narrower than the proximal and distal bodies; and wherein the proximal body, the intermediate body, and the proximal end of the distal body are positioned within the distal end of the lumen of the shaft.

2. The transeptal apparatus of claim 1, the tip member further including a body (432), the at least one fluid passageway including a recess (328) formed in a side portion of the body, optionally the recess including a distal portion and a proximal portion, the distal portion being positioned distally in relation to the distal end of the shaft, the proximal portion being positioned proximally in relation to the distal end of the shaft.

3. The transeptal apparatus of claim 2, the at least one fluid passageway including at least two recesses angularly spaced apart from each other equidistantly about a central longitudinal axis extending through the body.

4. The transeptal apparatus of claim 2 or claim 3, the recess extending inwardly toward a central longitudinal axis extending through the body.

5. The transeptal apparatus of claim 1, the proximal body comprising a disc shape.

6. The transeptal apparatus of any of claims 1 to 5, the at least one fluid passageway including a plurality of openings formed through the proximal body, the openings being angularly spaced apart from each other about a central longitudinal axis extending through the proximal body.

7. The transeptal apparatus of any of claims 1 to 6, the distal tip comprising a dome shape.

## Patentansprüche

1. Transseptaleinrichtung, umfassend:
(a) eine Körperanordnung;
(b) einen Schaft (410), der sich distal von der Körperanordnung erstreckt, wobei der Schaft einschließt:
(i) ein distales Ende und
(ii) ein Lumen (412); und
(c) ein Spitzenelement (420), das an dem distalen Ende des Schafts befestigt ist, wobei das Spitzenelement und das distale Ende des Schafts bemessen und konfiguriert sind, um in eine Kammer eines Herzens eines menschlichen Subjekts hinein zu passen, wobei das Spitzenelement einschließt:
(i) einen distalen Körper (430), umfassend:
eine distale Spitze (434), wobei die distale Spitze konfiguriert ist, um elektrische Energie an Gewebe abzugeben; und
ein proximales Ende (436);
(ii) einen proximalen Körper (450), der mit dem distalen Körper gekoppelt ist;
(iii) einen intermediären Körper (440), der zwischen dem proximalen Körper und dem distalen Körper zwischengelagert ist;
(iv) mindestens einen Fluiddurchgang (454) in Fluidverbindung mit dem Lumen des Schafts, wobei mindestens ein Abschnitt des mindestens einen Fluiddurchgangs des Spitzenelements proximal in Bezug auf das distale Ende des Schafts positioniert ist;
wobei der proximale Körper an dem Schaft befestigt ist, wobei der mindestens eine Fluiddurchgang durch den proximalen Körper hindurch ausgebildet ist;
das Lumen umfassend einen Innendurchmesser (ID2), wobei der distale Körper einen ersten Außendurchmesser (OD4) definiert, der proximale Körper einen zweiten Außendurchmesser (OD5) definiert, der zweite Außendurchmesser bemessen ist, um dem Innendurchmesser zu entsprechen, der erste Außendurchmesser derart kleiner als der Innendurchmesser ist, dass der distale Körper einen Spalt (416) mit dem Schaft definiert, wobei der Spalt und der mindestens eine durch den proximalen Körper hindurch ausgebildete Fluiddurchgang konfiguriert sind, um zusammenzuwirken, um einen Weg für die Fluidverbindung zwischen dem Lumen und einem Bereich außerhalb des Spitzenelements bereitzustellen;
wobei der intermediäre Körper schmaler als der proximale und der distale Körper ist; und wobei der proximale Körper, der intermediäre Körper und das proximale Ende des distalen Körpers innerhalb des distalen Endes des Lumens des Schafts positioniert sind.

2. Transseptaleinrichtung nach Anspruch 1, wobei das Spitzenelement ferner einen Körper (432) einschließt, wobei der mindestens eine Fluiddurchgang eine Aussparung (328) einschließt, die in einem Seitenabschnitt des Körpers ausgebildet ist, optional wobei die Aussparung einen distalen Abschnitt und einen proximalen Abschnitt einschließt, wobei der distale Abschnitt distal in Bezug auf das distale Ende des Schafts positioniert ist, wobei der proximale Abschnitt proximal in Bezug auf das distale Ende des Schafts positioniert ist.

3. Transseptaleinrichtung nach Anspruch 2, wobei der mindestens eine Fluiddurchgang mindestens zwei Aussparungen einschließt, die äquidistant um eine sich durch den Körper hindurch erstreckende zentrale Längsachse herum winkelmäßig voneinander beabstandet sind.

4. Transseptaleinrichtung nach Anspruch 2 oder 3, wobei sich die Aussparung nach innen in Richtung einer sich durch den Körper hindurch erstreckenden zentralen Längsachse erstreckt.

5. Transseptaleinrichtung nach Anspruch 1, der proximale Körper umfassend eine Scheibenform.

6. Transseptaleinrichtung nach einem der Ansprüche 1 bis 5, wobei der mindestens eine Fluiddurchgang eine Vielzahl von Öffnungen einschließt, die durch den proximalen Körper hindurch ausgebildet sind, wobei die Öffnungen um eine sich durch den proximalen Körper erstreckende zentrale Längsachse herum winkelmäßig voneinander beabstandet sind.

7. Transseptaleinrichtung nach einem der Ansprüche 1 bis 6, die distale Spitze umfassend eine Kuppelform.

## Revendications

1. Appareil transseptal, comprenant :
(a) un ensemble corps ;
(b) une tige (410) s'étendant distalement à partir de l'ensemble corps, la tige comportant :
(i) une extrémité distale, et
(ii) une lumière (412) ; et
(c) un élément de bout (420) fixé au niveau de l'extrémité distale de la tige, l'élément de bout et l'extrémité distale de la tige étant dimensionnés et conçus pour s'ajuster au sein d'une cavité d'un cœur d'un sujet humain, l'élément de bout comportant :
(i) un corps distal (430) comprenant :
un bout distal (434), le bout distal étant configuré pour délivrer de l'énergie électrique à un tissu ; et
une extrémité proximale (436) ;
(ii) un corps proximal (450) accouplé au corps distal ;
(iii) un corps intermédiaire (440) interposé entre le corps proximal et le corps distal ;
(iv) au moins une voie de passage de fluide (454) en communication fluidique avec la lumière de la tige, au moins une partie de l'au moins une voie de passage de fluide de l'élément de bout étant positionnée proximalement par rapport à l'extrémité distale de la tige ;
le corps proximal étant fixé à la tige, l'au moins une voie de passage de fluide étant formée à travers le corps proximal ;
la lumière comprenant un diamètre interne (ID2), le corps distal définissant un premier diamètre externe (OD4), le corps proximal définissant un second diamètre externe (OD5), le second diamètre externe étant dimensionné pour correspondre au diamètre interne, le premier diamètre externe étant plus petit que le diamètre interne de telle sorte que le corps distal définit un espace (416) avec la tige, l'espace et l'au moins une voie de passage de fluide formée à travers le corps proximal étant conçus pour coopérer pour fournir une voie pour une communication fluidique entre la lumière et une région extérieure à l'élément de bout ;
le corps intermédiaire étant plus étroit que les corps proximal et distal ; et dans lequel le corps proximal, le corps intermédiaire et l'extrémité proximale du corps distal sont positionnés au sein de l'extrémité distale de la lumière de la tige.

2. Appareil transseptal selon la revendication 1, l'élément de bout comportant en outre un corps (432), l'au moins une voie de passage de fluide comportant un évidement (328) formé dans une partie latérale du corps, facultativement l'évidement comportant une partie distale et une partie proximale, la partie distale étant positionnée distalement par rapport à l'extrémité distale de la tige, la partie proximale étant positionnée proximalement par rapport à l'extrémité distale de la tige.

3. Appareil transseptal selon la revendication 2, l'au moins une voie de passage de fluide comportant au moins deux évidements espacés angulairement l'un de l'autre à égale distance autour d'un axe longitudinal central s'étendant à travers le corps.

4. Appareil transseptal selon la revendication 2 ou la revendication 3, l'évidement s'étendant vers l'intérieur en direction d'un axe longitudinal central s'étendant à travers le corps.

5. Appareil transseptal selon la revendication 1, le corps proximal comprenant une forme de disque.

6. Appareil transseptal selon l'une quelconque des revendications 1 à 5, l'au moins une voie de passage de fluide comportant une pluralité d'ouvertures formées à travers le corps proximal, les ouvertures étant espacées angulairement les unes des autres autour d'un axe longitudinal central s'étendant à travers le corps proximal.

7. Appareil transseptal selon l'une quelconque des revendications 1 à 6, le bout distal comprenant une forme de dôme.
